# EUROPEAN PATENT SPECIFICATION

(11) **EP 0 922 694 B1**
(45) Date of publication and mention of the grant of the patent: **08.12.2004**
(21) Application number: 98310194.0
(22) Date of filing: 11.12.1998
(51) Int. Cl.: C07C 303/22, C07C 303/44, C07C 309/42, C07C 231/12

(54) **Preparation of esters**
Verfahren zur Herstellung von Estern
Procédé pour la préparation d'esters

(30) Priority: 11.12.1997 GB 9726247
(43) Date of publication of application: 16.06.1999
(73) Proprietor: THE ASSOCIATED OCTEL COMPANY LIMITED, London W1X 6DT (GB)
(72) Inventor: Clarke, Paul D., The Associated Octel Co., Ltd., Ellesmere Road, South Wirral L65 3HF (GB); Head, Robert A., The Associated Octel Co., Ltd., Ellesmere Port, South Wirral L65 3HF (GB); Radley, Peter M., The Associated Octel Co., Ltd., Ellesmere Port, South Wirral L65 3HF (GB)
(74) Representative: Matthews, Derek Peter

(56) References cited:
- EP-A- 0 120 591
- WO-A-96/39378
- US-A- 5 466 840
- US-A- 5 523 434

## Description

The invention relates to an improved process for the preparation of phenol esters which have utility as bleach activators.

As a result of renewed interest in the use of phenolsulphonate esters as bleach activators, there has been a resurgence of interest in improved methods of producing them.

EP 148148 describes the reaction of anhydrous sodium phenol sulphonate with an alkanoyl halide at 90-200°C in the absence of a solvent.

EP 120591 describes a similar reaction but uses refluxing halocarbon solvents as the reaction medium. Additionally, 1.2-1.5 mole equivalents of alkanoyl halide are employed as are extended reaction times (20 hours) to effect the reaction.

EP 150532 describes the reaction of an acyl chloride with anhydrous disodium phenol sulphonate (approx. 1:1 mole ratio) in glyme as a solvent. Typical reaction yields are 60-80%.

EP 166571 describes a similar process but uses a 1.2-1.5 mole equivalent of acyl chloride to give products in isolated yields of 40-65%.

Improved yields are claimed in EP 220826 wherein certain linear acyl chlorides are reacted with sodium phenol sulphonate at 160-170°C over 3-4 hours. A 2:1 mole ratio is typically employed and the necessary recycle of unreacted acid chloride is demonstrated.

Process improvements are also claimed in EP 402048 wherein a phase transfer catalyst is used to effect the reaction of an acid chloride with a phenol sulphonate salt. Isolated yields are typically 60-70% based on the starting acid chloride employed.

EP 164786 describes the use of potassium salts of phenolsulphonic acid instead of the sodium salt to improve reaction yields although no quantitative data is provided.

US 5523434 describes two-stage reactions in which the amino acid halides are prepared in a first step using inorganic acid halides or oxalyl chloride.

The prior art discussed above clearly demonstrates that obtaining conversion factors greater than 70% using acyl chlorides and salts of phenol sulphonic acid is difficult to achieve. Thus, a number of alternative processes have also been taught.

EP 125641 teaches the use of transesterification to effect the desired reaction whereby phenol esters of aliphatic acids are reacted with hydroxybenzene sulphonate salts. Very large molar excesses of phenol esters are used (up to 5:1) resulting in isolated yields of only 20-30% based on the starting phenol ester employed.

US 4544503 describes the reaction of phenol sulphonate salts with CO and an olefin at >125°C and >500 psig in an inert atmosphere with a metal carbonyl catalyst. Moderate yields are given (^{~}60%), and products are highly coloured.

Sulphonation of an appropriate phenol ester is described in EP 165480 and EP 201222 wherein good yields (70-90%) are claimed by careful control of the SO₃ sulphonation conditions.

Alternatively, the use of a mixed anhydride using acetic anhydride is described in US 4735740.

Similarly, the preparation of 6-benzoylamidocaproyl oxybenzene sulphonate is described in US 4634551 whereby the acid is reacted with anhydrous sodium phenol sulphonate using trifluoroacetic anhydride. Isolated yield was 48%.

EP 153222 describes the use of a symmetric anhydride to effect the reaction, acetic anhydride is disclosed but this process suffers from the principal disadvantage of only giving a maximum yield of 50% based on the starting acid on a first-pass process. Recycling is necessary and this is described.

Finally, the use of the Schotten-Baumann technique in the preparation of esters of oxybenzene sulphonate salts is given in EP 294073. The only synthesis exemplified involves the reaction of sodium phenol sulphonate with benzoyl chloride to make sodium benzoyloxybenzene sulphonate. No quantitative data is provided.

Summarising these disclosures, it can be seen that, despite considerable prior art teaching, there remains significant problems in finding simple, high-yielding, low colour generating, cost-effective processes to make oxybenzene salts such as oxybenzene sulphonate salts.

We have now developed an improved process for the preparation of phenol esters of formula (I) 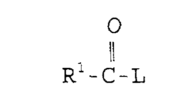 wherein R¹ is a linear or branched alkyl or alkenyl group of 3 to 20 carbon atoms optionally interrupted by -O- or -NH- and optionally substituted by an oxo group and L is a group of formula 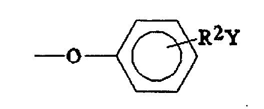 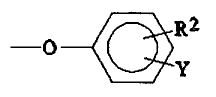 or 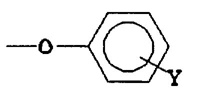 in which R² is an alkyl group or alkylene chain containing 1 to 8 carbon atoms and Y is a group -CO₂^{⊖}M^{⊕}, -SO₃^{⊖}M^{⊕} or -PO₄^{⊖}M^{⊕} in which M^{⊕} is a cation, preferably an alkali metal, ammonium or substituted ammonium cation. In particular M^{⊕} may be a sodium or potassium cation. It will be appreciated that the -O- or -NH- in R¹ can be adjacent to the oxo substituent thus forming an ester or amide linkage.

In a first aspect of the process according to the invention an acid chloride corresponding to R¹-CO- as defined above, if appropriate in an inert solvent, is reacted with a substituted phenol corresponding to L as defined above, in aqueous solution, in the presence of an inorganic base. At the end of the reaction the desired phenol ester is isolated by filtration in the presence of a flocculating agent, washing and drying. In the esterification process, an organic base, such as a pyridine or an N-alkylimidazole, is present as a catalyst.

The invention avoids the need for drying of the phenolsulphonate salt starting material, a process which presents considerable problems on scale-up, and allows use of commercially available phenolsulphonic acid solution.

Where the group R¹-CO- represents the residue of a 6-acylamidohexanoic acid, it can be prepared by the base hydrolysis of caprolactam in aqueous conditions (via either a batch or a continuous process), the resultant wet 6-aminohexanoic acid then being coupled with the relevant acid chloride under Schotten-Baumann conditions. The coupling product, 6-acylamidohexanoic acid, may be isolated by acidification and filtration or preferably may extracted into a suitable solvent for the next reaction stage. This represents a considerable improvement over the prior art in which such materials are prepared from 6-aminohexanoic acid itself.

The acid chloride corresponding to R¹-CO- may be prepared by the addition of 1 molar equivalent of thionyl chloride to the acid over a period of 10 minutes to 2 hours at room temperature. The acid may be suspended in a suitable solvent such as hexane, benzene, toluene, xylene, acetone, THF, DMF, sulpholane. Toluene is the preferred solvent and is used at a level of approximately 500 ml/mol of acid. Use of more than 1 equivalent of thionyl chloride leads to increased colour in the final product. After a suitable period (0.5-1 hours) the acid chloride solution is heated to a suitable temperature while being purged with nitrogen. Suitable temperatures are in the range 50-100°C, a temperature of 80°C being preferred. Such a heating period has the effect of driving off from the solution the acidic by-products from the acid chloride preparation which are held in the system at ambient temperatures. Failure to remove such by-products results in significantly reduced yields and a drop in assay of the isolated product.

The preparation of the acid chloride can be carried out by a batch or continuous process. In the case of a continuous reaction both the thionyl chloride and the solution of the acid are added to a stirred tank reactor at such a rate as to maintain the correct stoichiometry and give the desired residence time. The degassing stage is carried out in a second continuous reactor system.

An aqueous solution of the phenol salt containing about 1 molar equivalent of an inorganic base is prepared. Alternatively such a mixture can be prepared by the addition of 2 equivalents of base to an aqueous solution of the corresponding phenolic acid. This represents a considerable improvement over the prior art which employs a solid salt, for example a solid sulphonic acid salt. Suitable bases include the hydroxides of lithium, potassium, and sodium, sodium hydroxide being preferred. The solution also contains as catalyst an organic base such as a pyridine, preferably dimethylaminopyridine, or an N-alkylimidazole, preferably N-methylimidazole, at a loading of between 1-5 mol%, based on the amount of acid chloride to be charged. Absence of the catalyst can lead to markedly reduced yield. It is also beneficial for the solution to contain between 0.08-0.14% by weight of a non-ionic surfactant such as a polyethylene glycol. The presence of such a surfactant gives the product in a much more filterable form. The solution may contain up to 30% by weight of the phenol salt, for example about 10-11% by weight. More concentrated solutions lead to problems of agitation.

The stirred aqueous solution is then cooled to below 5°C. Use of higher temperatures can result in loss of yield due to hydrolysis of the product. The solution of the acid chloride is then added, typically over a period of between 1-3 hours. Sufficient acid chloride is prepared as to provide 1 molar equivalent of the acid chloride with respect to the phenol salt. This also represents a considerable improvement over the prior art in which relatively large excesses of the acid chloride are required in order to achieve moderate yields. The pH of the system is maintained at the desired level by the addition of an aqueous solution of the inorganic base. The pH should be held between 9-12, preferably at 10-10.5. Use of lower pH values gives much reduced yields. Higher pH values lead to an increased probability of hydrolysis of the product.

At the end of the addition period the system is stirred for a further period, preferably at below 5°C. Such a period can be from 30 minutes to 15 hours.

A further useful aspect of the process is the possibility of recovering unreacted acid R¹-COOH at the end of the reaction for recylcing and use as starting material. After filtering off the desired product the mother liquor is contacted with an inert solvent, for example one of those specified above and preferably toluene, heated and acidified. The acid R¹-COOH will partition into the inert solvent and can be used as an input material after reaction with thionyl chloride as described above.

It is evident that, on increasing the reaction scale, and where the power inputted by the agitator system is increased, the filtration characteristics become poorer leading to extended filtration times and (because of high moisture contents) unacceptably long drying times. Filtration is improved by addition to the product slurry solutions of flocculating agents such as the cationic polyelectrolytes based on polyacrylamide and its derivatives, i.e. polymers and copolymers of acrylamide, aminoalkylacrylamides and their *bis*-acrylamide analogues (to provide cross-linking), for example the Allied Colloids Zetag and Magnafloc ranges (Zetag and Magnafloc are trade marks), especially Zetag 89 and 87 and Magnafloc 1797. These are preferably added as a 0.1% aqueous solution at a loading of 200 ppm relative to the reaction mixture, prior to the filtration step.

The invention is particularly applicable to the synthesis of sodium (6-nonamidohexanoyl)oxybenzene sulphonate. The reaction mixture used in the preparation of this compound may contain a small amount of nonanoic acid, for example as a by-product of the production of the nonamidohexanoic acid starting material which need not be purified out before using the starting material.

### Example 1

6-Nonamidohexaonic acid (90.0 kg), and toluene (200L) were charged to a 100 gallon glass-lined Pfaudler reactor equipped with a beaver tail baffle containing a thermocouple pocket, a retreat 3 bladed turbine, a sparge pipe, a 50L vessel, and a condenser system. Thionyl chloride (39.0 kg) was added over 25 minutes at room temperature. The mixture was then stirred at room temperature for 1 hour. The resultant solution was then heated to 80°C for a further 1 hour with a nitrogen purge passing through the reaction mixture.

Sodium phenolsulphonate dihydrate (76.2 kg), 50% aqueous sodium hydroxide solution (27.0 kg), 4-dimethylaminopyridine (1.35 kg), and water (828L) were charged to a 300 gallon glass-lined Pfaudler reactor equipped with a beaver tail baffle containing a thermocouple pocket, a retreat 3 bladed turbine, a pH probe, two 100L pipette vessels, and a condenser system. The resultant solution was cooled to 2°C. The toluene solution of 6-nonamidohexanoyl chloride was then added over 3 hours at 0-5°C, the pH being maintained at 10-10.5 by the addition of 20% aqueous sodium hydroxide. The resultant slurry was stirred for a further 30 minutes at 0-5°C and pH 10-10.5.

A 0.1% aqueous solution of Zetag 89 (300L) was added over 4 minutes, the batch stirred for a further 5 minutes and the product was then filtered, washed with water (200L), and dried in vacuo to give 128.0 kg of material containing 122.5 kg of sodium (6-nonamidohexanoyl)oxybenzene sulphonate.

### Example 2

6-Nonamidohexanoic acid (15.0 kg), and toluene (33L) were charged to a 10 gallon glass-lined Pfaudler reactor equipped with a beaver tail baffle containing a thermocouple pocket, a retreat 3 bladed turbine, a sparge pipe, a 50L pipette vessel, and a condenser system. Thionyl chloride (6.50 kg) was added over 5 minutes at room temperature. The mixture was then stirred at room temperature for 1 hour. The resultant solution was then heated to 80°C for a further 1 hour with a nitrogen purge passing through the reaction mixture.

Sodium phenolsulphonate dihydrate (12.70 kg), 50% aqueous sodium hydroxide solution (4.50 kg), N-methylimidazole (0.192 kg), and water (138L) were charged to a 50 gallon glass-lined Pfaudler reactor equipped with a beaver tail baffle containing a thermocouple pocket, a retreat 3 bladed turbine, a pH probe, two 100L pipette vessels, and a condenser system. The resultant solution was cooled to 2°C. The toluene solution of 6-nonamidohexanoyl chloride was then added over 2 hours at 0-5°C, the pH being maintained at 10-10.5 by the addition of 20% aqueous sodium hydroxide. The resultant slurry was stirred for a further 30 minutes at 0-5°C and pH 10-10.5.

A 0.05% aqueous solution of Magnafloc 1797 (10L) was added over 2 minutes, the mixture stirred for 1 minute, a 0.1% aqueous solution of Zetag 89 (50L) was then added over 4 minutes, the batch stirred for a further 5 minutes and the product was then filtered, washed with water (50L), and dried in vacuo to give 23.0 kg of material containing 21.0 kg of sodium (6-nonamidohexanoyl)oxybenzene sulphonate.

### Example 3

Reactions were carried out at 0.15 molar scale with an agitator speed of 1500 rpm. At the end of the reaction aliquots were taken and flocculant added to give required dose. The flocculant addition was made at 1,500 rpm over 10 seconds, material stirred for a further 2 minutes at 1500 rpm and 2 minutes at 400 rpm. The material was filtered through a 7 cm Buchner funnel using grade 54 papers. Filtration time is the time for a cake to form, deliquour time is the time for liquour to then stop passing into receiver (<1 drop in 30 seconds). Wash time is the time for 20 ml water to stop passing through the undisturbed cake. Flocculant used in this example is Zetag 89.

| Run | Flocculant level (ppm) | Filtration time (seconds) | Deliquour time (minutes) | Wash time (minutes) |
|---|---|---|---|---|
| 1 | 200 | 15 | 4.5 | 6 |
| 2 | 200 | 18 | 5.5 | 6 |
| 3 | 200 | 16 | 5.5 | 9 |
| 4 | 100 | 15 | 6 | 6.5 |
| 5 | 100 | 20 | 7 | 5 |
| 6 | 50 | 70 | 9 | 11 |
| 7 | 50 | 71 | 7 | 5 |
| 8 | 0 | 690 | Note | 8 |
| Note: No clear time for formation of cake. Time included in filtration time. | | | | |

## Claims

1. A process for preparing a phenol ester of formula I 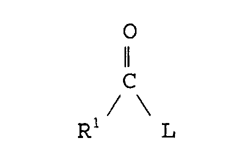 (wherein R¹ is a linear or branched alkyl or alkenyl group optionally interrupted by an -O- or -NH- group and optionally substituted by an oxo group and L is a group of formula 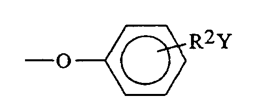 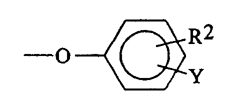 or 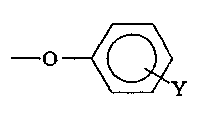 in which R² is an alkyl group or alkylene chain and Y is a group -CO₂⁻M⁺, SO₃⁻M⁺ or PO₄⁻M⁺ (wherein M is a cation)) comprising the step of reacting an acid chloride corresponding to R¹-CO- with a substituted phenol corresponding to L, in aqueous solution, in the presence of an inorganic base and as a catalyst an organic base, followed by filtration, **characterised in that** said filtration is in the presence of a flocculating agent.

2. A process as claimed in claim 1 wherein the flocculating agent is a cationic polyelectrolyte based on polyacrylamide and/or the derivatives thereof.

3. A process as claimed in claim 2 wherein the flocculating agent is a cationic (co-)polymer of acrylamide and/or one or more aminoalkylacrylamides and optionally one or more bis-acrylamide analogues thereof.

4. A process as claimed in any one of claims 1 to 3 wherein said organic base is selected from pyridine or an N-alkylimidazole.

5. A process as claimed in claim 4 wherein the catalyst is dimethylaminopyridine or N-methylimidazole.

6. A process as claimed in any of claims 1 to 5 in which the acid chloride and the substituted phenol are reacted in a substantially 1:1 molar ratio.

7. A process as claimed in any of claims 1 to 6 wherein the reaction is carried out at a pH of 9-12.

8. A process as claimed in any preceding claim wherein the inorganic base is lithium, sodium or potassium hydroxide.

9. A process as claimed in any preceding claim wherein the acid chloride is prepared by reaction of the corresponding acid with thionyl chloride.

10. A process as claimed in any preceding claim wherein the aqueous solution of the substituted phenol is prepared by addition of 2 equivalents of inorganic base to an aqueous solution of the corresponding phenolic acid.

11. A process as claimed in any preceding claim in which a non-ionic surfactant is present in the reaction medium.

12. A process as claimed in any preceding claim wherein R¹CO- is a 6-acylamidohexanoyl group.

13. A process as claimed in claim 12 wherein the 6-acylamidohexanoic acid corresponding to the 6-acylamidohexanoyl group is prepared by base hydrolysis of caprolactam and coupling of the resultant 6-aminohexanoic acid with an acyl chloride.

14. A process as claimed in claim 12 or claim 13 wherein the compound of formula (I) is sodium 6-(nonamidohexanoyl)-oxybenzene sulphonate.

## Patentansprüche

1. Verfahren zur Herstellung eines Phenolesters der Formel I 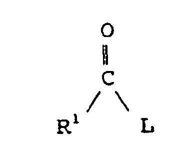 (worin R¹ für eine lineare oder verzweigte Alkyl- oder Alkenylgruppe steht, die gegebenenfalls durch eine -O- oder -NH-Gruppe unterbrochen und gegebenenfalls durch eine Oxogruppe substituiert ist, und L für eine Gruppe der Formel 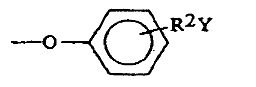 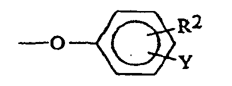 oder 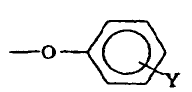 steht, worin R² für eine Alkylgruppe oder Alkylenkette steht und Y für eine Gruppe -CO₂⁻M⁺, SO₃⁻M⁺ oder PO₄⁻M⁺ steht (worin M für ein Kation steht)), bei dem man ein R¹-CO- entsprechendes Säurechlorid mit einem L entsprechenden substituierten Phenol in wässriger Lösung in Gegenwart einer anorganischen Base und eines Katalysators in Form einer organischen Base umsetzt, und anschließend filtriert, **dadurch gekennzeichnet, dass** man in Gegenwart eines Flockungsmittels filtriert.

2. Verfahren nach Anspruch 1, wobei es sich bei dem Flockungsmittel um einen kationischen Polyelektrolyten auf der Basis von Polyacrylamid und/oder den Derivaten davon handelt.

3. Verfahren nach Anspruch 2, wobei es sich bei dem Flockungsmittel um ein kationisches (Co)polymer von Acrylamid und/oder einem oder mehreren Aminoalkylacrylamiden und gegebenenfalls einem oder mehreren Bisacrylamidanalogen davon handelt.

4. Verfahren nach einem der Ansprüche 1 bis 3, wobei die organische Base unter Pyridin oder einem N-Alkylimidazol ausgewählt ist.

5. Verfahren nach Anspruch 4, wobei es sich bei dem Katalysator um Dimethylaminopyridin oder N-Methylimidazol handelt.

6. Verfahren nach einem der Ansprüche 1 bis 5, bei dem man das Säurechlorid und das substituierte Phenol in einem molaren Verhältnis von im Wesentlichen 1:1 umsetzt.

7. Verfahren nach einem der Ansprüche 1 bis 6, wobei die Umsetzung bei einem pH von 9 bis 12 erfolgt.

8. Verfahren nach einem der vorhergehenden Ansprüche, wobei es sich bei der anorganischen Base um Lithium-, Natrium- oder Kaliumhydroxid handelt.

9. Verfahren nach einem der vorhergehenden Ansprüche, wobei man das Säurechlorid durch Umsetzung der entsprechenden Säure mit Thionylchlorid herstellt.

10. Verfahren nach einem der vorhergehenden Ansprüche, wobei man die wässrige Lösung des substituierten Phenols durch Zugabe von 2 Äquivalenten anorganischer Base zu einer wässrigen Lösung der entsprechenden Phenolsäure herstellt.

11. Verfahren nach einem der vorhergehenden Ansprüche, wobei im Reaktionsmedium ein nichtionisches Tensid vorliegt.

12. Verfahren nach einem der vorhergehenden Ansprüche, wobei R¹CO- für eine 6-Acylamidohexanoyl-Gruppe steht.

13. Verfahren nach Anspruch 12, wobei man die der 6 Acylamidohexanoyl-Gruppe entsprechende 6-Acylamidohexansäure durch basische Hydrolyse von Caprolactam und Kupplung der erhaltenen 6-Aminohexansäure mit einem Acylchlorid herstellt.

14. Verfahren nach Anspruch 12 oder 13, wobei es sich bei der Verbindung der Formel (I) um Natrium-6-(nonamidohexanoyl)-oxybenzolsulfonat handelt.

## Revendications

1. Procédé de préparation d'un phénol ester de formule I 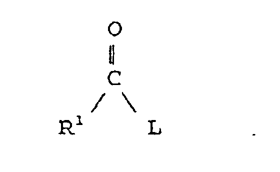 (dans laquelle R¹ est un groupe alcoyle ou alcényle, linéaire ou ramifié, interrompu éventuellement par un groupe - O - ou - NH - et substitué éventuellement par un groupe oxo et L est un groupe de formules 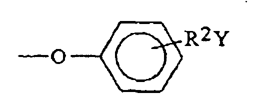 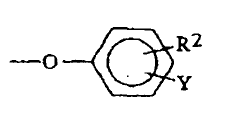 ou 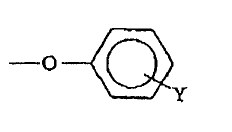 dans lesquelles R² est un groupe alcoyle ou une chaîne alcoylène et Y est un groupe -CO₂⁻M⁺, SO₃⁻M⁺ ou PO₄⁻M⁺ (dans lesquelles M est un cation)) comprenant le stade de réaction d'un chlorure d'acide correspondant à R¹-CO- sur un phénol substitué correspondant à L, en solution aqueuse, en la présence d'une base minérale et comme catalyseur d'une base organique, suivie d'une filtration, **caractérisé en ce que** la filtration s'effectue en la présence d'un agent de floculation.

2. Procédé suivant la revendication 1, dans lequel l'agent de floculation est un polyélectrolyte cationique à base de polyacrylamide et/ou de ses dérivés.

3. Procédé suivant la revendication 2, dans lequel l'agent de floculation est un (co) polymère cationique d'acrylamide et/ou d'un ou de plusieurs aminoalcoylacrylamides et éventuellement d'un ou de plusieurs de ses analogues bis-acrylamide.

4. Procédé suivant l'une quelconque des revendications 1 à 3, dans lequel la base organique est choisie parmi la pyridine ou un N-alcoylimidazole.

5. Procédé suivant la revendication 4, dans lequel le catalyseur est la diméthylaminopyridine ou le N-méthyl-imidazole.

6. Procédé suivant l'une quelconque des revendications 1 à 5, dans lequel le chlorure d'acide et le phénol substitués sont mis à réagir en un rapport molaire de sensiblement 1 : 1.

7. Procédé suivant l'une quelconque des revendications 1 à 6, dans lequel on effectue la réaction à un pH de 9 à 12.

8. Procédé suivant l'une quelconque des revendications précédentes, dans lequel la base minérale est l'hydroxyde de lithium, l'hydroxyde de sodium ou l'hydroxyde de potassium.

9. Procédé suivant l'une quelconque des revendications précédentes, dans lequel on prépare le chlorure d'acide en faisant réagir l'acide correspondant sur du chlorure de thionyle.

10. Procédé suivant l'une quelconque des revendications précédentes, dans lequel on prépare la solution aqueuse du phénol substitué par addition de 2 équivalents d'une base minérale à une solution aqueuse de l'acide phénolique correspondant.

11. Procédé suivant l'une quelconque des revendications précédentes, dans lequel un agent tensioactif non-ionique est présent dans le milieu de réaction.

12. Procédé suivant l'une quelconque des revendications précédentes dans lequel R¹CO- est un groupe 6-acylamidohexanoyle.

13. Procédé suivant la revendication 12 dans lequel on prépare l'acide 6-acylamidohexanoique correspondant au groupe 6-acylamidohexanoyle par hydrolyse basique de caprolactame et couplage de l'acide 6-aminohexanoique obtenu avec un chlorure d'acyle.

14. Procédé suivant la revendication 12 ou revendication 13, dans lequel le composé de formule (I) est le 6-(nonamidohexanoyl)-oxybenzène sulfonate de sodium.
